(19) Europäisches Patentamt
European Patent Office
Office européen des brevets

(11) Veröffentlichungsnummer: **0 069 848**
**A2**

# (12) EUROPÄISCHE PATENTANMELDUNG

(21) Anmeldenummer: 82104532.5

(22) Anmeldetag: 25.05.82

(51) Int. Cl.[3]: C 07 D 249/08
C 07 D 249/10, C 07 D 233/60
C 07 D 231/12, C 07 D 231/16
C 07 D 231/18, A 01 N 43/50
A 01 N 43/56, A 01 N 43/64

(30) Priorität: 04.06.81 DE 3122174

(43) Veröffentlichungstag der Anmeldung:
19.01.83 Patentblatt 83/3

(84) Benannte Vertragsstaaten:
AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Förster, Heinz, Dr.
Am Eckbusch 47
D-5600 Wuppertal 1(DE)

(72) Erfinder: Eue, Ludwig, Dr.
Paul-Klee-Strasse 36
D-5090 Leverkusen 1(DE)

(72) Erfinder: Schmidt, Robert, Dr.
Im Waldwinkel 110
D-5060 Bergisch-Gladbach 2(DE)

(72) Erfinder: Lürssen, Klaus, Dr.
August-Kierspel-Strasse 89
D-5060 Bergisch-Gladbach 2(DE)

## (54) Substituierte Phenoxyphenyl-azolylalkylether, Verfahren zu ihrer Herstellung sowie ihre Verwendung als Herbizide und Pflanzenwuchsregulatoren.

(57) Substituierte Phenoxyphenyl-azolylalkylether der Formel

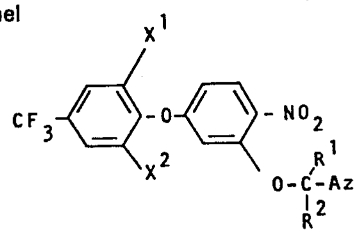

(I)

in welcher

$X^1$ und $X^2$ unabhängig voneinander für Wasserstoff oder Halogen stehen,

$R^1$ und $R^2$ unabhängig voneinander für Wasserstoff oder Alkyl mit 1 bis 4 Kohlenstoffatomen stehen und

Az für einen gegebenenfalls substituierten Azolylrest stehen,

sowie deren Säureadditions-Salze, Metallsalz-Komplexe und quaternäre Alkylierungsmittel-Additionssalze, mehrere Verfahren zu deren Herstellung und deren Verwendung als Herbizide und als Pflanzenwuchsregulatoren.

Verbindung der Formel

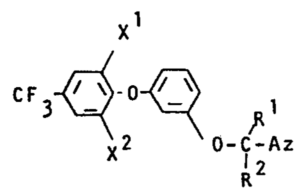

(IV)

in welcher

$X^1$, $X^2$, $R^1$, $R^2$ und Az die oben angegebene Bedeutung haben,

sowie ein Verfahren zu deren Herstellung.

EP 0 069 848 A2

Croydon Printing Company Ltd.

BAYER AKTIENGESELLSCHAFT 5090 Leverkusen, Bayerwerk

Zentralbereich
Patente, Marken und Lizenzen Dü/ABc
Ib

# Substituierte Phenoxyphenyl-azolylalkylether, Verfahren zu ihrer Herstellung sowie ihre Verwendung als Herbizide und Pflanzenwuchsregulatoren

Die Erfindung betrifft neue substituierte Phenoxyphenyl-azolylalkylether, mehrere Verfahren zu deren Herstellung sowie deren Verwendung als Herbizide und Pflanzenwuchsregulatoren.

Es ist bereits bekannt geworden, daß bestimmte Phenoxyphenylether herbizide Eigenschaften besitzen (vgl. DE-OS 2 311 638 und US-PS 4 093 446). So läßt sich zum Beispiel α-(5-(2-Chlor-4-trifluormethyl-phenoxy)-2-nitro-phenoxy)-propionsäureethylester zur Bekämpfung von Unkraut verwenden. Die Wirkung dieses Stoffes ist gut, jedoch ist die Selektivität nicht immer ausreichend.

Es wurden nun neue substituierte Phenoxyphenylazolylalkylether der Formel

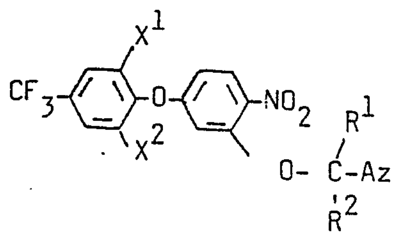

(I)

in welcher

$X^1$ und $X^2$ unabhängig voneinander für Wasserstoff oder Halogen stehen,

$R^1$ und $R^2$ unabhängig voneinander für Wasserstoff oder Alkyl mit 1 bis 4 Kohlenstoffatomen stehen und

Az für einen gegebenenfalls substituierten Azolylrest steht,

sowie deren Säureadditions-Salze, Metallsalz-Komplexe und quaternäre Alkylierungsmittel-Additionssalze

gefunden.

Weiterhin wurde gefunden, daß man die neuen substituierten Phenoxyphenyl-azolyl-alkylether der Formel (I), deren Säureadditions-Salze, Metallsalz-Komplexe und quaternäre Alkylierungsmittel-Additionssalze erhält, wenn man

(a) substituierte Phenoxyphenole der Formel

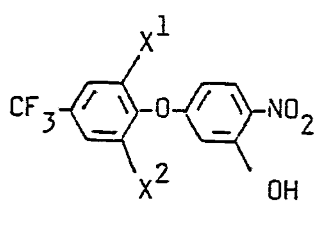

(II)

in welcher

$X^1$ und $X^2$ die oben angegebene Bedeutung haben,

mit Halogenalkyl-azolen der Formel

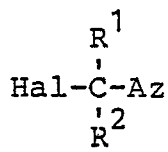

(III)

in welcher

$R^1$, $R^2$ und Az die oben angegebene Bedeutung haben und

Hal für Chlor oder Brom steht,

gegebenenfalls in Gegenwart eines Säureakzeptors und gegebenenfalls in Gegenwart eines Verdünnungsmittels umsetzt, oder

(b) substituierte Phenoxyphenyl-azolylalkylether der Formel

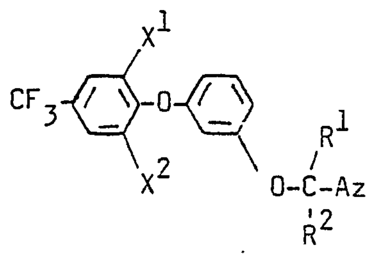

(IV)

in welcher

$X^1$, $X^2$, $R^1$, $R^2$ und Az die oben angegebene Bedeutung haben,

mit Salpetersäure, gegebenenfalls in Gegenwart eines Katalysators und gegebenenfalls eines Verdünnungsmittels umsetzt,

und gegebenenfalls anschließend an die so erhaltenen substituierten Phenoxyphenyl-azolyl-alkylether der

Formel (I) eine Säure, ein Metallsalz oder ein Alkylierungsmittel addiert.

Schließlich wurde gefunden, daß sich die neuen substituierten Phenoxyphenylazolylalkylether sowie deren Säureadditions-Salze, Metallsalz-Komplexe und deren quaternäre Alkylierungsmittel-Additionssalze durch hervorragende herbizide und pflanzenwuchsregulierende Eigenschaften auszeichnen.

Überraschenderweise besitzen die erfindungsgemäßen substituierten Phenoxyphenylazolylalkylether der Formel (I) sowie deren Säureadditions-Salze, Metallsalz-Komplexe und deren quaternäre Alkylierungsmittel-Additionssalze eine wesentlich bessere selektive herbizide Wirksamkeit als der α-(5-(2-Chlor-4-trifluormethyl-phenoxy)-2-nitrophenoxy)-propionsäureethylester, welches ein konstitutionell ähnlicher, vorbekannter Wirkstoff gleicher Wirkungsrichtung ist. Darüber hinaus lassen sich die erfindungsgemäßen Stoffe auch als Pflanzenwuchsregulatoren einsetzen. Vor allem können sie als Defoliants und Desiccants bei Baumwolle verwendet werden.

Die erfindungsgemäßen substituierten Phenoxyphenyl-azolylalkylether sind durch die Formel (I) allgemein definiert.

In dieser Formel stehen vorzugsweise

$X^1$ für Chlor,
$X^2$ für Wasserstoff oder Chlor,
$R^1$ und $R^2$ unabhängig voneinander für Wasserstoff

oder Alkyl mit 1 oder 2 Kohlenstoffatomen und

Az für einen über ein Ring-Stickstoffatom gebundenen Pyrazolyl-, Imidazol-yl-, 1,2,4-Triazolyl-, 1,2,3-Triazolyl- oder 1,3,4-Triazolyl-rest, wobei jeder dieser Azolyl-Reste ein- oder mehrfach, gleichartig oder verschieden substituiert sein kann durch Fluor, Chlor, Brom, Jod, Alkyl mit 1 bis 4 Kohlenstoffatomen, (welches seinerseits gegebenenfalls durch Fluor, Chlor, Cyano, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen und/oder Alkoxycarbonyl mit 1 bis 4 Kohlenstoffatomen in der Alkoxygruppe substituiert ist,) Alkoxy mit 1 bis 4 Kohlenstoffatomen, Alkylthio mit 1 bis 4 Kohlenstoffatomen, Cyano, Carboxy, Alkoxycarbonyl mit 1 bis 4 Kohlenstoffatomen in der Alkoxygruppe, Alkylcarbonyl mit 1 bis 4 Kohlenstoffatomen in der Alkylgruppe und/oder Phenyl, welches seinerseits gegebenenfalls durch Halogen, Alkyl mit 1 bis 4 Kohlenstoffatomen, Trifluormethyl, Cyano, Nitro und/oder Alkoxy mit 1 bis 4 Kohlenstoffatomen substituiert ist.

Besonders bevorzugt sind diejenigen erfindungsgemäßen substituierten Phenoxyphenylazolylalkylether der Formel (I), in denen

$X^1$ für Chlor steht,
$X^2$ für Wasserstoff oder Chlor steht,

$R^1$ und $R^2$ unabhängig voneinander für Wasserstoff oder Methyl stehen und

Az für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein-, zwei- oder dreifach, gleichartig oder verschieden substituiert sein kann durch Fluor, Chlor, Brom, Alkyl mit 1 bis 2 Kohlenstoffatomen, welches seinerseits gegebenenfalls durch Fluor, Chlor, Cyano, Alkoxy mit 1 oder 2 Kohlenstoffatomen, Alkylthio mit 1 oder 2 Kohlenstoffatomen, Alkylsulfonyl mit 1 oder 2 Kohlenstoffatomen und/oder Alkoxycarbonyl mit 1 bis 2 Kohlenstoffatomen in der Alkoxygruppe substituiert ist,) Alkoxy mit 1 oder 2 Kohlenstoffatomen, Alkylthio mit 1 oder 2 Kohlenstoffatomen, Cyano, Carboxy, Alkoxycarbonyl mit 1 oder 2 Kohlenstoffatomen in der Alkoxygruppe, Alkylcarbonyl mit 1 bis 2 Kohlenstoffatomen in der Alkylgruppe und/oder Phenyl, welches seinerseits gegebenenfalls durch Fluor, Chlor, Brom, Alkyl mit 1 oder 2 Kohlenstoffatomen, Trifluormethyl, Cyano, Nitro und/oder Alkoxy mit 1 oder 2 Kohlenstoffatomen substituiert ist.

Bevorzugt sind außerdem Säureadditions-Salze von substituierten Phenoxyphenyl-azolyl-alkylethern der Formel (I),

in denen $X^1$, $X^2$, $R^1$, $R^2$ und Az die vorzugsweise genannten Bedeutungen haben. Besonders bevorzugt sind dabei solche Säureadditions-Salze, die durch Addition von Halogenwasserstoffsäure, wie zum Beispiel Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie zum Beispiel Essigsäure, Maleinsäure, Bernsteinsäure, Fumarsäure, Weinsäure, Zitronensäure, Salicylsäure, Sorbinsäure, Milchsäure, sowie Sulfonsäuren, wie zum Beispiel p-Toluolsulfonsäure und 1,5-Naphthalindisulfonsäure entstehen.

Bevorzugt sind auch Metallsalz-Komplexe von substituierten Phenoxyphenyl-azolyl-alkylethern der Formel (I), in denen $X^1$, $X^2$, $R^1$, $R^2$ und Az die vorzugsweise genannten Bedeutungen haben. Besonders bevorzugt sind dabei solche Metallsalz-Komplexe, die als Kationen Metalle der II. bis IV. Hauptgruppe oder der I. und II. oder IV. bis VIII. Nebengruppe des Periodensystems der Elemente enthalten, wobei Kupfer, Zink, Mangan, Magnesium, Zinn, Eisen und Nickel beispielhaft genannt seien.

Anionen dieser Metallsalz-Komplexe sind vorzugsweise solche, die sich von Halogenwasserstoffsäuren, insbe- Chlorwasserstoffsäure und Bromwasserstoffsäure, ferner von Phosphorsäure, Salpetersäure und Schwefelsäure ableiten.

Bevorzugt sind schließlich auch von substituierten Phen-

oxyphenyl-azolylalkylethern der Formel (I), in denen $X^1$, $X^2$, $R^1$, $R^2$ und Az die vorzugsweise genannten Bedeutungen haben, abgeleitete quaternäre Salze mit Alkylierungsmitteln.

Besonders bevorzugt sind dabei solche quaternären Salze, die durch Addition von Alkylhalogeniden und Alkylsulfaten entstehen. Insbesondere in Frage kommen Alkylchloride, -bromide und -iodide mit jeweils 1 bis 4 Kohlenstoffatomen, ferner Dialkylsulfate mit 1 bis 4 Kohlenstoffatomen pro Alkylgruppe.

Verwendet man 5-(2,6-Dichlor-4-trifluormethyl-phenoxy)-2-nitro-phenol und 1-Brommethyl-pyrazol als Ausgangsstoffe, so läßt sich der Verlauf des erfindungsgemäßen Verfahrens (a) durch das folgende Formelschema wiedergeben:

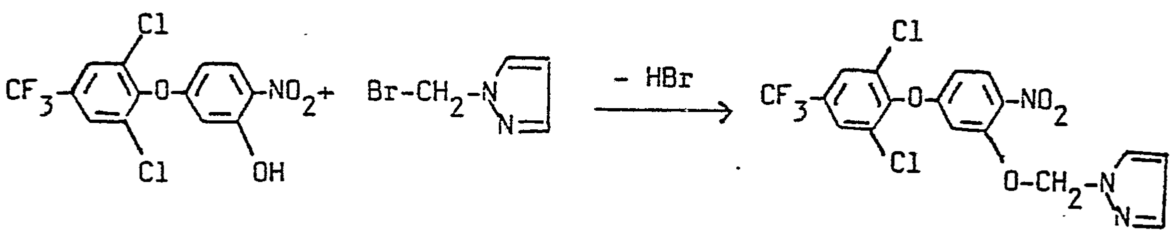


Verwendet man 3-(2-Chlor-4-trifluor-methyl-phenoxy)-phenyl-1-pyrazolyl-methylether als Ausgangsstoff und Salpetersäure als Nitrierungsmittel, so läßt sich der Verlauf des erfindungsgemäßen Verfahrens (b) durch das folgende Formelschema wiedergeben:

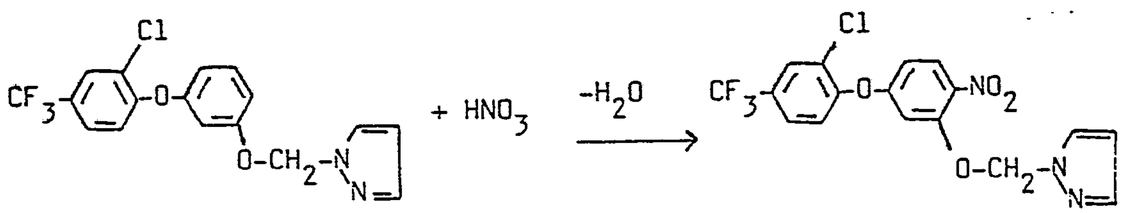

Die bei dem erfindungsgemäßen Verfahren (a) als Ausgangsstoffe benötigten substituierten Phenoxyphenole sind durch die Formel (II) allgemein definiert. In dieser Formel haben $X^1$ und $X^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele seien 5-(2-Chlor-4-trifluormethyl-phenoxy)-2-nitro-phenol und 5-(2,6-Dichlor-4-trifluormethyl-phenoxy)-2-nitro-phenol genannt.

Die Verbindungen der Formel (II) sind bereits bekannt (vgl. US-PS 3 928 416).

Die bei dem Verfahren (a) weiterhin als Ausgangsstoffe zu verwendenden Halogenalkylazole sind durch die Formel (III) allgemein definiert. In dieser Formel haben $R^1$, $R^2$ und Az vorzugsweise diejenigen Bedeutungen, die bereits im Zusammenhang mit der Beschreibung der erfindungsgemäßen Stoffe der Formel (I) vorzugsweise für diese Reste genannt wurden. Hal steht in der Formel (III) für Chlor oder Brom.

Als Beispiele für die Verbindungen der Formel (III) seien genannt: 1-Brommethyl-pyrazol, 1-Chlormethyl-pyrazol, 1-(1-Chlorethyl)-pyrazol, 1-Chlormethyl-imidazol, 1-Chlormethyl-1,2,4-triazol, 1-Chlormethyl-1,3,4-triazol, 1-Chlormethyl-3,5-dimethyl-pyrazol, 1-Chlormethyl-2-methylimidazol, 1-Chlormethyl-3-methyl-pyrazol, 3-Chlor-1-chlor-methyl-pyrazol, 1-Chlormethyl-4-methyl-pyrazol,

1-Chlormethyl-4-methoxy-pyrazol, 1-Chlormethyl-3-methylthio-pyrazol und 4-Chlor-1-chlormethyl-3,5-dimethyl-pyrazol.

Die Verbindungen der Formel (III) sind bereits bekannt (vgl. DE-OS 2 835 147 und 2 835 158).

Die bei dem erfindungsgemäßen Verfahren (b) als Ausgangsstoffe benötigten substituierten Phenoxyphenyl-azolylalkylether sind durch die Formel (IV) definiert.

In dieser Formel haben $X^1$, $X^2$, $R^1$, $R^2$ und Az vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für die Verbindungen der Formel (IV) seien genannt: 3-(2-Chlor-4-trifluormethyl-phenoxy)-phenyl- und 3-(2,6-Dichlor-4-trifluormethyl-phenoxy)-phenyl-1-pyrazolylmethylether, -1-(1-pyrazolylethyl)-ether, -1-imidazolylmethylether, -1-(1,2,4-triazolyl)-methylether, -1-(1,3,4-triazolyl)-methylether, -1-(3,5-dimethyl-pyrazolyl)-methylether, -1-(2-methyl-imidazolyl)-methylether, -1-(3-chlor-1,2,4-triazolyl)-methylether, -1-(4-chlor-pyrazolyl)-methylether, -1-(4-methyl-pyrazolyl)-methylether, -1-(4-methoxy-pyrazolyl)-methylether, -1-(3-methylthio-pyrazolyl)-methylether und -1-(4-chlor-3,5-dimethyl—pyrazolyl)-methylether.

Die Verbindungen der Formel (IV) sind noch nicht in der Literatur beschrieben; sie lassen sich jedoch nach an sich bekannten Verfahren in einfacher Weise herstellen. Man erhält sie beispielsweise, wenn man substituierte Phenoxyphenole der Formel

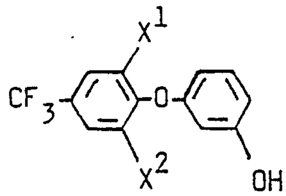

(V)

in welcher

$X^1$ und $X^2$ die oben angegebene Bedeutung haben,

mit Halogenalkyl-azolen der Formel

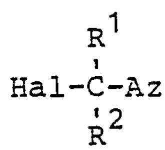

(III)

in welcher

$R^1$, $R^2$, Az und Hal die oben angegebene Bedeutung haben,

gegebenenfalls in Gegenwart eines Säureakzeptors und gegebenenfalls in Gegenwart eines Verdünnungsmittels umsetzt.

Die bei dem obigen Verfahren als Ausgangsstoffe benötigten substituierten Phenoxyphenole sind durch die Formel (V) definiert. In dieser Formel haben $X^1$ und $X^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Beispiele für die Verbindungen der Formel (V) sind 3-(2-Chlor-4-trifluormethyl-phenoxy)-phenol und 3-(2,6-Dichlor-4-trifluormethyl-phenoxy)-phenol. Die Verbindungen der Formel (V) sind bereits bekannt (vgl. DE-OS 2 805 981).

Bei dem obigen Verfahren entsprechen die Umsetzungsbedingungen im einzelnen denjenigen des erfindungsgemäßen Verfahrens (a).

Das erfindungsgemäße Verfahren (a ) wird vorzugsweise in Gegenwart eines Verdünnungsmittels durchgeführt. Als derartige Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methyl-isobutylketon, außerdem Ester, wie Essigsäure-methylester und -ethylester, ferner Nitrile, wie z.B. Acetonitril und Propionitril, darüber hinaus Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Als Säureakzeptoren können bei dem erfindungsgemäßen Verfahren (a) alle üblichen Säurebindemittel verwendet werden. Hierzu gehören vorzugsweise Alkalicarbonate und Alkalialkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperaturen können bei dem erfindungsgemäßen Verfahren (a) innerhalb eines größeren Bereiches variiert werden. Im allgemeinen arbeitet man bei Temperaturen zwischen -20 und +100°C, vorzugsweise zwischen 0 und 80°C.

Das erfindungsgemäße Verfahren (a) wird im allgemeinen unter Normaldruck durchgeführt.

Zur Durchführung des erfindungsgemäßen Verfahrens (a) werden die Ausgangsstoffe der Formeln (II) und (III) in angenähert äquimolaren Mengen eingesetzt. Ein größerer Überschuß der einen oder anderen Komponente bringt keine größeren Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Die Aufarbeitung wird nach üblichen Methoden durchführt. Bei Raumtemperatur kristalline Produkte erhält man durch Verdünnen mit Wasser und Absaugen.

Bei einer anderen Aufarbeitungsweise wird mit Wasser und einem mit Wasser praktisch nicht mischbaren organischen Lösungsmittel, wie z. B. Toluol versetzt, geschüttelt, die organische Phase abgetrennt, mit Wasser gewaschen, getrocknet, filtriert und eingeengt, wobei man das Produkt der Formel (I) als Rückstand erhält.

Das erfindungsgemäße Verfahren (b) wird gegebenenfalls unter Verwendung eines Verdünnungsmittels durchgeführt. Als Verdünnungsmittel kommen hierbei vorzugsweise halogenierte Kohlenwasserstoffe, wie z.B. Methylenchlorid, in Betracht.

Das erfindungsgemäße Verfahren (b) wird gegebenenfalls in Gegenwart eines Katalysators durchgeführt. Als Katalysatoren kommen vorzugsweise Protonensäuren, wie z.B. Schwefelsäure oder Essigsäure, in Betracht.

Die Reaktionstemperatur wird bei Verfahren (b) im allgemeinen zwischen -2o und +8o°C, vorzugsweise zwischen 0 und 5o°C gehalten. Verfahren (b) wird im allgemeinen bei Normaldruck durchgeführt.

Zur Durchführung von Verfahren (b) setzt man auf 1 Mol substituierten Phenoxyphenyl-azolyl-alkylether der Formel (IV) im allgemeinen 0,9 bis 2 Mol, vorzugsweise 1,0 bis 1,3 Mol Salpetersäure und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt.

Die Aufarbeitung wird nach üblichen Methoden, beispielsweise durch Eingießen in Eiswasser, Absaugen und gegebenenfalls Umkristallisieren, oder wie oben für Verfahren (a) beschrieben, durchgeführt.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen von Verbindungen der Formel (I) kommen vorzugsweise Salze von denjenigen Anionen und Kationen in Betracht, die bereits im Zusammenhang mit der Beschreibung der erfindungsgemäßen Metallsalz-Komplexe als bevorzugt genannt wurden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisation reinigen.

Zur Herstellung von quaternären Salzen von erfindungsgemäßen Verbindungen der Formel (I) mit Alkylierungsmitteln kommen insbesondere diejenigen Alkylierungsmittel in Frage, die bereits im Zusammenhang mit der Beschreibung der erfindungsgemäßen Stoffe als besonders bevorzugte Alkylierungsmittel genannt wurden.

Die quaternären Alkylierungsmittel-Additionssalze von Verbindungen der Formel (I) können in einfacher Weise nach üblichen Verfahren erhalten werden. So lassen sie

sich zum Beispiel herstellen, indem man Verbindungen der Formel (I) mit Alkylierungsmitteln in Gegenwart eines inerten organischen Verdünnungsmittels, wie Acetonitril, umsetzt.

Die kristallin anfallenden Stoffe können durch Abfiltrieren isoliert und gegebenenfalls durch Waschen mit einem inerten organischen Lösungsmittel gereinigt werden.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe sind besonders gut zur selektiven Unkrautbekämpfung in verschiedenen Kulturen, wie zum Beispiel Mais und Baumwolle, geeignet.

Die erfindungsgemäßen Wirkstoffe besitzen außerdem eine sehr gute pflanzenwachstumsregulierende Wirksamkeit. Sie eignen sich besonders gut zur Wuchshemmung, sowie zur Entlaubung und Austrocknung der Blätter bei Baumwolle.

Die Wirkstoffe können in die üblichen Formulierungen überführt werden, wie Lösungen, Emulsionen, Spritzpulver, Suspensionen, Pulver, Stäubemittel, lösliche Pulver, Granulate, Suspensions-Emulsionskonzentrate, Wirkstoffimprägnierte Natur- und synthetische Stoffe, Feinstverkapselungen in polymeren Stoffen und Aerosole.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid, als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden oder Pflanzenwachstumsregulatoren zur Unkrautbekämpfung bzw. als Pflanzenwuchsregulatoren Verwendung finden, wobei Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Eumulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei einer Anwendung als Herbizide sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Für die Anwendung der erfindungsgemäßen Wirkstoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die angewandte Wirkstoffmenge kann in einem größeren Bereich schwanken. Sie hängt im wesentlichen ab von der Art des gewünschten Effektes. Im allgemeinen liegen die Aufwandmengen zwischen o,ol und 5o kg Wirkstoff pro Hektar Bodenfläche, vorzugsweise zwischen o,o5 und lo kg pro ha.

Die Herstellung und die Verwendung der erfindungsgemäßen Wirkstoffe geht aus den nachfolgenden Beispielen hervor.

Herstellungsbeispiele

Beispiel 1

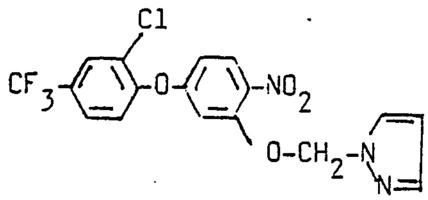

12,2 g (o,o75-Mol) 1-Chlormethyl-pyrazol werden zu einer auf 3o°C erwärmten Mischung aus 25,o g 5-(2-Chlor-4-trifluormethyl-phenoxy)-2-nitro-phenol, 24 g Kaliumcarbonat und 12o ml Aceton gegeben. Das Gemisch wird 8 Stunden bei 5o bis 55°C gerührt. Dann wird mit Wasser verdünnt, mit Toluol extrahiert, die organische Phase mit Wasser gewaschen, getrocknet, filtriert und eingeengt. Man erhält 2o,9 g (67% der Theorie) (5-(2-Chlor-4-trifluormethyl-phenoxy)-phenyl)-(1-pyrazolylmethyl)-ether vom Schmelzpunkt 75°C als hellgelben, kristallinen Rückstand.

Nach der im Beispiel 1 angegebenen Methode oder auch nach der Verfahrensvariante (b) werden die in der nachstehenden Tabelle formelmäßig aufgeführten Verbindungen hergestellt.

Tabelle 1

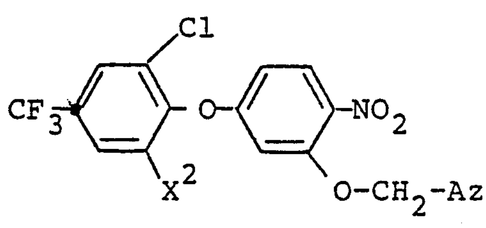

(Ia)

0069848



Tabelle 1

| Beispiel Nr. | $X^2$ | Az | Schmelzpunkt (°C) |
|---|---|---|---|
| 2 | Cl | 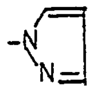 | 111 |
| 3 | H | 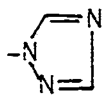 | |
| 4 | Cl | 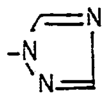 | |
| 5 | H | 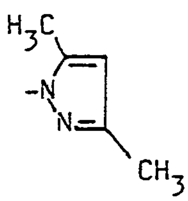 | |
| 6 | Cl | 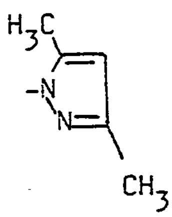 | |
| 7 | H | 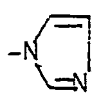 | |
| 8 | Cl | 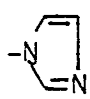 | |

Tabelle 1 (Fortsetzung)

| Beispiel Nr. | $X^2$ | Az | Schmelzpunkt (°C) |
|---|---|---|---|
| 9 | H | 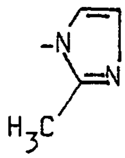 | |
| 1o | Cl | 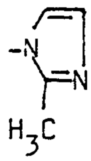 | |
| 11 | H | 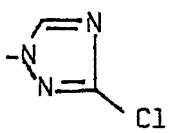 | |
| 12 | Cl | 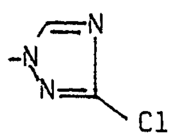 | |
| 13 | H | 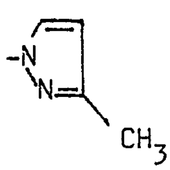 | |
| 14 | Cl | 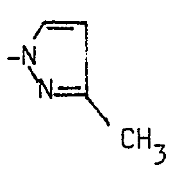 | |

Tabelle 1 (Fortsetzung)

| Beispiel Nr. | $X^2$ | Az | Schmelzpunkt (°C) |
|---|---|---|---|
| 15 | H | 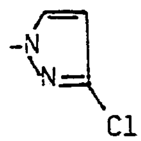 | |
| 16 | Cl | 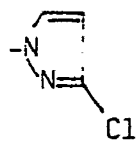 | |
| 17 | H | 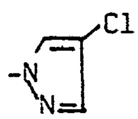 | |
| 18 | Cl | 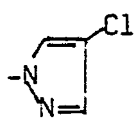 | |
| 19 | H | 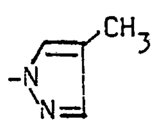 | |
| 2o | Cl | 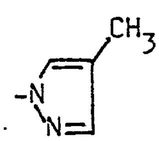 | |
| 21 | H | 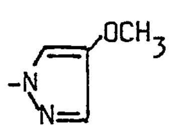 | |

Tabelle 1 (Fortsetzung)

| Beispiel Nr. | $X^2$ | Az | Schmelzpunkt (°C) |
|---|---|---|---|
| 22 | Cl | 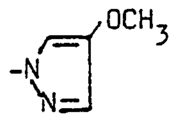 | |
| 23 | H | 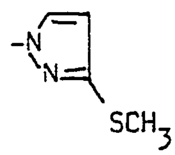 | |
| 24 | Cl | 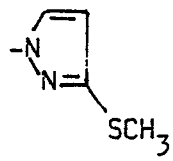 | |
| 25 | H | 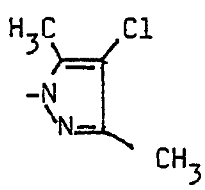 | |
| 26 | Cl | 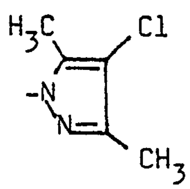 | |

In den folgenden Verwendungsbeispielen wird die nachstehend angegebene Verbindung als Vergleichssubstanz eingesetzt:

(A) =

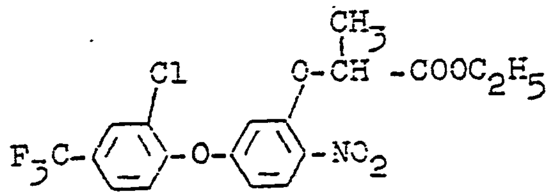

(bekannt aus DE-OS 2 311 638 bzw. US-PS 4 093 446)
α-(5-(2-Chlor-4-trifluormethyl-phenoxy)-2-nitrophenoxy)-propionsäureethylester.

Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (1) und (2) eine bessere selektive herbizide Wirksamkeit als die Vergleichssubstanz (A).

Beispiel B

Entlaubung und Austrocknung der Blätter bei Baumwolle

Lösungsmittel: 3o Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
+ leichtes Austrocknen der Blätter, geringer Blattfall
++ starkes Austrocknen der Blätter, starker Blattfall
+++ sehr starkes Austrocknen der Blätter, sehr starker Blattfall

In diesem Test zeigen die erfindungsgemäßen Stoffe (1) und (2) eine gute Wirksamkeit.

Formulierungsbeispiel

Zur Herstellung eines Spritzpulvers werden

| | | |
|---|---|---|
| 50 | Gewichtsteile | Wirkstoff gemäß Beispiel 1, |
| 2 | " | Netzmittel |
| 3 | " | Dispergiermittel auf Basis von Ligninsulfat |
| 5 | " | Alkylarylsulfonat |
| 3 | " | Reisstärke |
| 1 | " | Calgon |
| 20 | " | hochdisperse Kieselsäure |
| 16 | " | Kaolin |

in einem Lödige-Mischer intensiv gemischt und anschliessend in einem "Mikronizer 8" fein gemahlen. Das erhaltene Pulver wird vor der Ausbringung auf die Pflanzen in der jeweils gewünschten Menge mit Wasser vermischt.

Patentansprüche

1. Substituierte Phenoxyphenyl-azolylalkylether der Formel

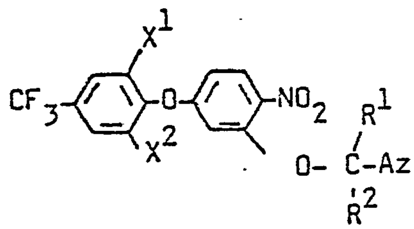

(I)

in welcher

$X^1$ und $X^2$ unabhängig voneinander für Wasserstoff oder Halogen stehen,

$R^1$ und $R^2$ unabhängig voneinander für Wasserstoff oder Alkyl mit 1 bis 4 Kohlenstoffatomen stehen und

Az für einen gegebenenfalls substituierten Azolylrest steht,

sowie deren Säureadditions-Salze, Metallsalz-Komplexe und quaternäre Alkylierungsmittel-Additionssalze.

2. Substituierte Phenoxyphenyl-azolylalkylether der Formel (I), in denen $X^1$ für Chlor steht, $X^2$ für Wasserstoff oder Chlor steht, $R^1$ und $R^2$ unabhängig voneinander für Wasserstoff oder Alkyl mit 1 oder 2 Kohlenstoffatomen stehen und Az für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein- oder mehrfach gleichartig oder verschieden substituiert sein kann durch Fluor, Chlor, Brom, Jod, Alkyl mit 1 bis 4 Kohlenstoffatomen, (welches seinerseits gegebenenfalls durch Fluor, Chlor, Cyano, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen und/oder Alkoxycarbonyl mit 1 bis 4 Kohlenstoffatomen in der Alkoxygruppe substituiert ist,) Alkoxy mit 1 bis 4 Kohlenstoffatomen, Alkylthio mit 1 bis 4 Kohlenstoffatomen, Cyano, Carboxy, Alkoxycarbonyl mit 1 bis 4 Kohlenstoffatomen in der Alkoxygruppe, Alkylcarbonyl mit 1 bis 4 Kohlenstoffatomen in der Alkylgruppe und/oder Phenyl, welches seinerseits gegebenenfalls durch Halogen, Alkyl mit 1 bis 4 Kohlenstoffatomen, Trifluormethyl, Cyano, Nitro und/oder Alkoxy mit 1 bis 4 Kohlenstoffatomen substituiert ist, sowie deren Säureadditions-Salze, Metallsalz-Komplexe und quaternäre Alkylierungsmittel-Additionssalze.

3. Verfahren zur Herstellung von substituierten Phenoxyphenyl-azolylalkylethern der Formel

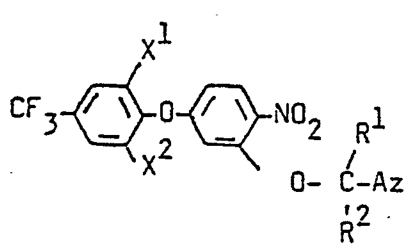

(I)

in welcher
$X^1$ und $X^2$ unabhängig voneinander für Wasserstoff oder Halogen stehen,

$R^1$ und $R^2$ unabhängig voneinader für Wasserstoff oder Alkyl mit 1 bis 4 Kohlenstoffatomen stehen und

Az für einen gegebenenfalls substituierten Azolylrest steht,

sowie deren Säureadditions-Salzen, Metallsalz-Komplexen und quaternären Alkylierungsmittel-Additionssalzen, dadurch gekennzeichnet, daß man

(a) substituierte Phenoxyphenole der Formel

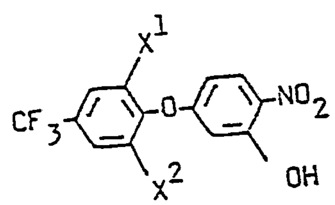

(II)

in welcher
$X^1$ und $X^2$ die oben angegebene Bedeutung haben,

mit Halogenalkyl-azolen der Formel

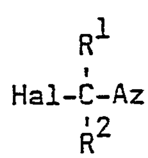

(III)

in welcher
$R^1$, $R^2$ und Az die oben angebene Bedeutung haben
und
Hal für Chlor oder Brom steht,

gegebenenfalls in Gegenwart eines Säureakzeptors und gegebenenfalls in Gegenwart eines Verdünnungsmittels umsetzt, oder

(b) substituierte Phenoxyphenyl-azolylalkylether der Formel

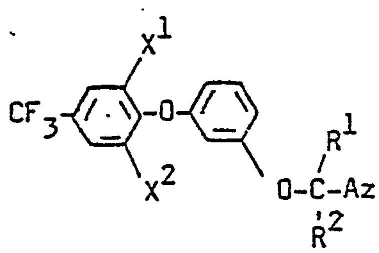

(IV)

in welcher
$X^1$, $X^2$, $R^1$, $R^2$ und Az die oben angegebene Bedeutung haben,

mit Salpetersäure, gegebenenfalls in Gegenwart eines Katalysators und gegebenenfalls in Gegenwart eines Verdünnungsmittels umsetzt,

und gegebenenfalls anschließend an die so erhaltenen substituierten Phenoxyphenyl-azolylalkylether der Formel (I) eine Säure, ein Metallsalz oder ein Alkylierungsmittel addiert.

4. Herbizide und pflanzenwuchsregulierende Mittel, gekennzeichnet durch einen Gehalt an mindestens einem substituiertem Phenoxyphenyl-azolylalkylether der Formel (I) bzw. einem von deren Säureadditions-Salzen, Metallsalz-Komplexen oder quaternären Alkylierungsmittel-Additionssalzen.

5. Verfahren zur Bekämpfung von Unkräutern sowie zur Regulierung des Pflanzenwachstums, dadurch gekennzeichnet, daß man substituierte Phenoxyphenyl-azolylalkylether der Formel (I) bzw. deren Säureadditions-Salze, Metallsalz-Komplexe bzw. deren quaternäre Alkylierungsmittel-Additionssalze auf die Unkräuter bzw. die zu regulierenden Pflanzen und/oder deren Lebensraum ausbringt.

6. Verwendung von substituierten Phenoxyphenyl-azolylalkylethern der Formel (I) bzw. von deren Säureadditions-Salzen, Metallsalz-Komplexen und quaternären Alkylierungsmittel-Additionssalzen zur Bekämpfung von Unkräutern sowie zur Regulierung des Pflanzenwachstums.

7. Substituierte Phenoxyphenyl-azolylalkylether der Formel

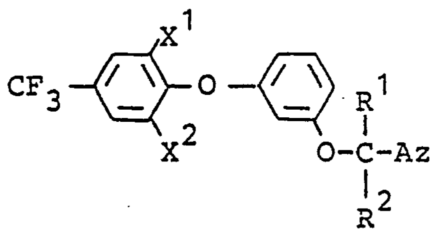

(IV)

in welcher

$X^1$ und $X^2$ unabhängig voneinander für Wasserstoff oder Halogen stehen,

$R^1$ und $R^2$ unabhängig voneinander für Wasserstoff oder Alkyl mit 1 bis 4 Kohlenstoffatomen stehen und

Az für einen gegebenenfalls substituierten Azolylrest steht.

8. Verfahren zur Herstellung von substituierten Phen-oxyphenyl-azolylalkylethern der Formel

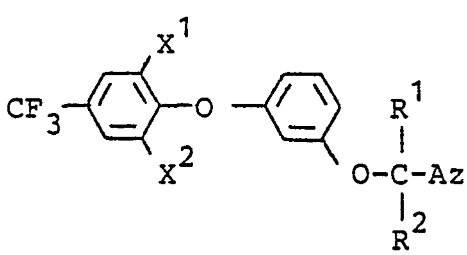

(IV)

in welcher
$X^1$ und $X^2$ unabhängig voneinander für Wasserstoff oder Halogen stehen,

$R^1$ und $R^2$ unabhängig voneinander für Wasserstoff oder Alkyl mit 1 bis 4 Kohlenstoffatomen stehen und

Az für einen gegebenenfalls substituierten Azolylrest steht,

dadurch gekennzeichnet, daß man substituierte Phenoxyphenole der Formel

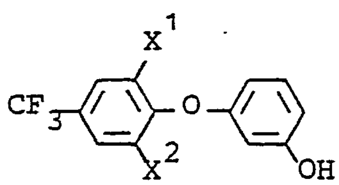

(V)

in welcher
$X^1$ und $X^2$ die oben angegebene Bedeutung haben,

mit Halogenalkyl-azolen der Formel

$$\text{Hal-}\overset{R^1}{\underset{R^2}{C}}\text{-Az} \qquad \text{(III)}$$

in welcher
$R^1$, $R^2$, Az und Hal die oben angegebene Bedeutung haben,

gegebenenfalls in Gegenwart eines Säureakzeptors und gegebenenfalls in Gegenwart eines Verdünnungsmittels umsetzt.

9. Substituierter Phenoxyphenyl-azolylalkylether der Formel

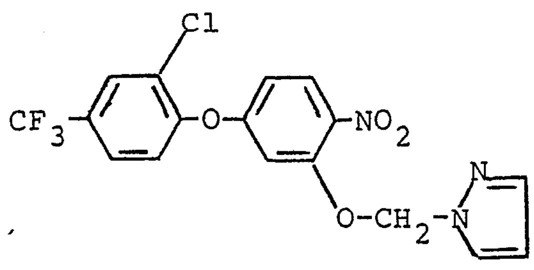

10. Substituierter Phenoxyphenyl-azolylalkylether der Formel